# EUROPEAN PATENT APPLICATION

(11) **EP 3 800 173 A1**
(43) Date of publication of application: **07.04.2021**
(21) Application number: 19201107.0
(22) Date of filing: 02.10.2019
(51) Int. Cl.: C07C 41/14, C07C 43/16, C08F 16/32, C08F 116/18, C03C 25/10, C08F 16/26

(54) **VINYL-ETHERS AND METHODS OF THEIR PRODUCTION**

(71) Applicant: Henkel AG & Co. KGaA, 40589 Düsseldorf (DE)
(72) Inventor: BRANDT, Adrian, 45219 Essen (DE); BECK,Horst, 41470 Neuss (DE); SPIEGELBERG, Brian, 18057 Rostock (DE); DE VRIES, Johannes Gerardus, 18055 Rostock (DE)
(74) Representative: Viering, Jentschura & Partner mbB Patent- und Rechtsanwälte

(57) **Abstract**

The present invention relates to methods for producing at least one vinyl ether compound of formula (I) and to a vinyl ether compound of formula (I) preferably obtainable by the methods according to the invention. Furthermore, the present invention relates to a polymer obtainable by polymerizing the vinyl ether compound of formula (I) according to the invention, to an adhesive comprising the at least one polymer according to the invention and to the use of at least one vinyl ether compound of formula (I) according to the invention or at least one polymer according to the invention for the production of UV adhesives, cationic curings or 1-component or 2-component systems.

## Description

The present invention relates to methods for producing at least one vinyl ether compound of formula (I) and to a vinyl ether compound of formula (I) preferably obtainable by the methods according to the invention. Furthermore, the present invention relates to a polymer obtainable by polymerizing the vinyl ether compound of formula (I) according to the invention, to an adhesive comprising the at least one polymer according to the invention and to the use of at least one vinyl ether compound of formula (I) according to the invention or at least one polymer according to the invention for the production of UV adhesives, cationic curing or 1-component or 2-component systems.

UV adhesives and reactive adhesives are widely used in the art and are versatile adhesives used for many different applications, including but not limited to automotive industry, furniture, veneers, electronics and the like. Most of the existing processes for producing compounds suitable for this type of adhesive are not based on sustainable and environmentally friendly techniques.

Thus, there is need in the art, for alternative manufacturing processes that can overcome the known drawbacks and provide compounds in economically sufficient yield, based on renewable resources for sustainable adhesive systems with a good performance.

Therefore, the present invention is based on the inventors' finding that mono- and divinyl ether compounds are obtainable by a specific method starting from, preferably at least one bio-based substrate, in the presence of a catalyst and optionally a bidentate ligand and/or a base. It was found that the ratio of produced mono- and divinyl ether compounds, as well as the ratio of primary and secondary mono vinyl ether compounds, can be influenced by the reaction temperature.

In a first aspect, the present invention relates to a method for producing at least one vinyl ether compound of formula (I) 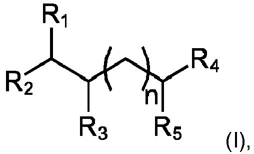 wherein
n is an integer of 0 to 5;
R₁, R₃ and R₄ are independently selected from -OH, -O-CH=CH₂, -H or a substituted or unsubstituted C₁-C₁₀ alkyl or C₂-C₁₀ alkenyl group; and
R₂ and R₅ are independently selected from -H or a substituted or unsubstituted C₁-C₁₀ alkyl or C₂-C₁₀ alkenyl group;
with the proviso that at least two of R₁, R₃ and R₄ are -OH or -O-CH=CH₂, and wherein at least one of R₁, R₃ and R₄ is -O-CH=CH₂;
wherein the method comprises or consists of the following steps:
reacting a compound of formula (II) 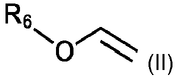 wherein R₆ is -C(=O)R₁₂, or an unsubstituted C₁-C₁₀ alkyl or C₂-C₁₀ alkenyl group; and R₁₂ is an unsubstituted C₁-C₁₀ alkyl group;
with a compound of formula (III) 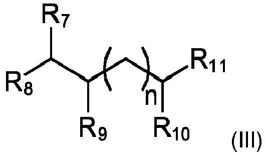 wherein
n is an integer of 0 to 5;
R₇, R₉ and R₁₁ are independently selected from -OH, -H or a substituted or unsubstituted C₁-C₁₀ alkyl or C₂-C₁₀ alkenyl group; and
R₈ and R₁₀ are independently selected from -H or a substituted or unsubstituted C₁-C₁₀ alkyl or C₂-C₁₀ alkenyl group;
with the proviso that at least two of R₇, R₉ and R₁₁ are -OH;
in the presence of a catalyst and optionally a bidentate ligand and/or a base.

In various embodiments, in the compound of formula (I)
n is 1; and/or
R₁ is -OH or -O-CH=CH₂; and/or
R2 is -CH₃; and/or
R3 is -H; and/or
R₄ is -OH or -O-CH=CH₂; and/or
R₅ is -H;
wherein at least one of R₁ and R₄ is -O-CH=CH₂, preferably R₁ and R₄ are -O-CH=CH₂; and/or
wherein R₆ in the compound of formula (II) is an unsubstituted C₂ alkyl group; and/or
wherein in formula (III)
n is 1; and/or
R₇ is -OH; and/or
R₈ is -CH₃; and/or
R₉ is -H; and/or
R₁₀ is -H; and/or
R₁₁ is -OH.

In one embodiment, in the compound of formula (II), R₆ is a C₁-C₆ alkyl group, most preferably a C2 alkyl group.

In various embodiments, the reaction temperature of the method according to the invention is between 20 to 90 °C, preferably between 50 to 80°C or 30 to 60 °C.

In various embodiments of the method according to the invention,
(i) the catalyst is selected from Pd catalysts, preferably Pd(acac)₂, Pd(dba)₂, Pd(OAc)₂, Pd(NO₃)₂*xH₂O, Pd(PPh₃)₄, PdCl₂(PPh₃)₂, Pd(pivalate) or Pd(II) trifluoroacetate, more preferably Pd(II) trifluoroacetate; and/or
(ii) the bidentate ligand is selected from phenanthrolines and bipyridines, preferable 2,2'-bipyridine; and/or
(iii) the base is a tertiary amine, preferably triethylamine.

In addition, the method according to the invention can be carried out
(i) in the presence of at least one solvent, preferably a polar solvent, more preferably tetrahydrofuran (THF), ethyl acetate (EtOAc), acetone, 1,4-dioxane or acetonitrile; or
(ii) without any additional solvent; and/or
(ii) under oxygen or air.

In various embodiments of the method according to the invention, the compound of formula (II) is added in molar excess over the compound of formula (III), preferably in a molar equivalent ratio of at least 1:1, >1:1, 2:1 or 3:1, for example at least 5:1, more preferably of at least 10:1, more preferably of at least 15:1 (formula (II):formula (III)).

In further embodiments of the method according to the invention, the vinyl ether compound of formula (I) is a mixture of divinyl ether and mono vinyl ether compounds, preferably the mixture comprises at least 50 wt.-% divinyl ether, more preferably at least 60 wt.-%, more preferably at least 70 wt.-%, most preferably at least 80 wt.-%, based on the total weight of the mixture.

In a second aspect, the present invention is a method for producing at least one vinyl ether compound of formula (I) 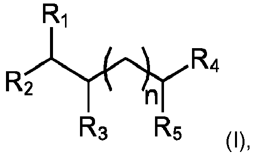 wherein
n is an integer of 0 to 5;
R₁, R₃ and R₄ are independently selected from -OH, -O-CH=CH₂, -H or a substituted or unsubstituted C₁-C₁₀ alkyl or C₂-C₁₀ alkenyl group; and
R₂ and R₅ are independently selected from -H or a substituted or unsubstituted C₁-C₁₀ alkyl or C₂-C₁₀ alkenyl group;
with the proviso that at least two of R₁, R₃ and R₄ are -OH or -O-CH=CH₂, and wherein at least one of R₁, R₃ and R₄ is -O-CH=CH₂;
wherein the method comprises or consists of the following steps:
reacting a compound of formula (III) 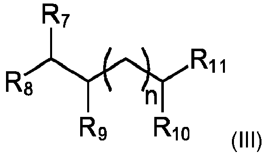 wherein
n is an integer of 0 to 5;
R₇, R₉ and R₁₁ are independently selected from -OH, -H or a substituted or unsubstituted C₁-C₁₀ alkyl or C₂-C₁₀ alkenyl group; and
R₈ and R₁₀ are independently selected from -H or a substituted or unsubstituted C₁-C₁₀ alkyl or C₂-C₁₀ alkenyl group;
with the proviso that at least two of R₇, R₉ and R₁₁ are -OH;
with
(i) acetylene in the presence of a catalyst and under pressure; or
(ii) calcium carbide, water and a base.

In various embodiments, in the compound of formula (I):
n is 1; and/or
R₁ is -OH or -O-CH=CH₂; and/or
R2 is -CH₃; and/or
R3 is -H; and/or
R₄ is -OH or -O-CH=CH₂; and/or
R₅ is -H;
wherein at least one of R₁ and R₄ is -O-CH=CH₂, preferably R₁ and R₄ are -O-CH=CH₂; and/or
wherein in formula (III)
n is 1; and/or
R₇ is -OH; and/or
R₈ is -CH₃; and/or
R₉ is -H; and/or
R₁₀ is -H; and/or
R₁₁ is -OH.

In some embodiments of aspect 1 and 2, the compound of formula (III) is bio-based.

In a third aspect, the present invention relates to a vinyl ether compound of formula (I) 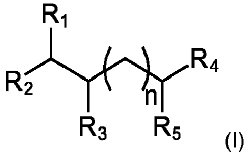 wherein
n is an integer of 0 to 5;
R₁, R₃ and R₄ are independently selected from -OH, -O-CH=CH₂, -H or a substituted or unsubstituted C₁-C₁₀ alkyl or C₂-C₁₀ alkenyl group; and
R₂ and R₅ are independently selected from -H or a substituted or unsubstituted C₁-C₁₀ alkyl or C₂-C₁₀ alkenyl group;
with the proviso that at least two of R₁, R₃ and R₄ are -OH or -O-CH=CH₂, and wherein at least one of R₁, R₃ and R₄ is -O-CH=CH₂;
preferably obtained by a method according to the invention.

In preferred embodiments, the vinyl ether compound is of formula (IV) 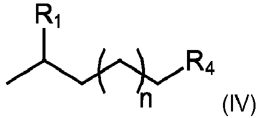 wherein
n is 0, 1, 2, 3, 4 or 5, preferably 1, 2 or 3, most preferably 1;
R₁ and R₄ are -OH or -O-CH=CH₂, with the proviso that at least one of R₁ and R₄ is -O-CH=CH₂, preferably R₁ and R₄ are -O-CH=CH₂; or
the vinyl ether compound is any one of the compounds of formulae (V) to (VII) 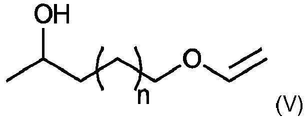 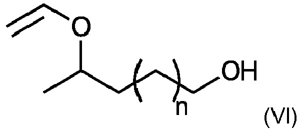 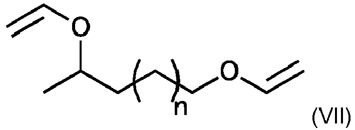 or a mixture thereof;
wherein
n is 0, 1, 2, 3, 4 or 5, preferably 1, 2 or 3, most preferably 1.

In a fourth aspect, the present invention relates to a polymer obtainable by polymerizing a vinyl ether compound of formula (I) according to the invention optionally together with at least one comonomer.

In a fifth aspect, the present invention relates to an adhesive comprising at least one polymer according to the invention.

Finally, in a sixth aspect, the present invention relates to a use of at least one vinyl ether compound of formula (I) according to the invention or at least one polymer according to the invention for the production of UV adhesives, cationic curings, or 1-component or 2-component systems, e.g. can coatings, epoxy- and acrylate-based coatings, structural adhesives, sealants, battery adhesives, wood coatings or pressure sensitive adhesives (PSA)).

The details of one or more embodiments are set forth in the accompanying drawings and the description below. Other features, objects, and advantages will be apparent from the description, drawings and examples, and from the claims.

The following detailed description refers to, by way of illustration, specific details and embodiments in which the invention may be practiced. These embodiments are described in sufficient detail to enable those skilled in the art to practice the invention. Other embodiments may be utilized and structural and logical changes may be made without departing from the scope of the invention. The various embodiments are not necessarily mutually exclusive, as some embodiments can be combined with one or more other embodiments to form new embodiments.

Unless otherwise defined, all technical and scientific terms used herein have the same meaning as commonly understood by one of ordinary skill in the art. The singular terms "a", "an" and "the" include plural referents unless context clearly indicates otherwise.

"At least one", as used herein, means one or more, i.e. 1, 2, 3, 4, 5, 6, 7, 8, 9 or more of the referenced species. Similarly, "one or more", as used herein, relates to at least one and comprises 1, 2, 3, 4, 5, 6, 7, 8, 9 or more. In connection with a given species, the term does not relate to the total number of molecules, but rather to the type of species, e.g. vinyl ether compounds. In connected with amounts, the term relates to the total amount of the referenced species.

Numeric values specified without decimal places refer to the full value specified with one decimal place, i.e. for example, 99 % means 99.0 %, unless otherwise defined.

The terms "about" or "approximately" or "approx.", in connection with a numerical value, refer to a variance of ±10 %, preferably ±5 %, more preferably ±2 %, more preferably ±1 %, and most preferably less than ±1 %, with respect to the given numerical value.

When an amount, a concentration or other values or parameters is/are expressed in form of a range, a preferable range, or a preferable upper limit value and a preferable lower limit value, it should be understood as that any ranges obtained by combining any upper limit or preferable value with any lower limit or preferable value are specifically disclosed, without considering whether the obtained ranges are clearly mentioned in the context.

According to the first aspect, the present invention relates to a method for producing at least one vinyl ether compound of formula (I) 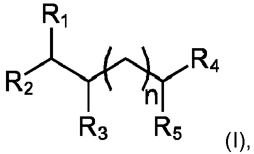 wherein
n is an integer of 0 to 5, preferably 1, 2 or 3, most preferably 1;
R₁, R₃ and R₄ are independently selected from -OH, -O-CH=CH₂, -H or a substituted or unsubstituted C₁-C₁₀ alkyl or C₂-C₁₀ alkenyl group; and
R₂ and R₅ are independently selected from -H or a substituted or unsubstituted C₁-C₁₀ alkyl or C₂-C₁₀ alkenyl group;
with the proviso that at least two of R₁, R₃ and R₄ are -OH or -O-CH=CH₂, and wherein at least one of R₁, R₃ and R₄ is -O-CH=CH₂, preferably R₁ and/or R₄ is/are -O-CH=CH₂;
wherein the method comprises or consists of the following steps:
reacting a compound of formula (II) 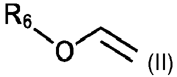
wherein R₆ is -C(=O)R₁₂, or an unsubstituted C₁-C₁₀ alkyl or C₂-C₁₀ alkenyl group, preferably an unsubstituted C₁-C₆ alkyl group, most preferably an unsubstituted C₂ alkyl group; and
R₁₂ is an unsubstituted C₁-C₁₀ alkyl group, preferably -CH₃,
with a compound of formula (III) 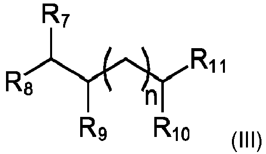 wherein
n is an integer of 0 to 5, preferably 1, 2 or 3, most preferably 1;
R₇, R₉ and R₁₁ are independently selected from -OH, -H or a substituted or unsubstituted C₁-C₁₀ alkyl or C₂-C₁₀ alkenyl group; and
R₈ and R₁₀ are independently selected from -H or a substituted or unsubstituted C₁-C₁₀ alkyl or C₂-C₁₀ alkenyl group;
with the proviso that at least two of R₇, R₉ and R₁₁ are -OH, preferably R₇ and R₁₁ are -OH;
in the presence of a catalyst and optionally a bidentate ligand and/or a base.

In various embodiments of the method according to the invention, the compound of formula (III) is bio-based.

In general, unless otherwise stated, bio-based materials or biomaterials include or are preferably made of materials, chemicals and energy derived from renewable biological resources, for example from living organisms such as plants, bacteria or animals, without being limited to these. "Bio-based", as used in this context, thus refers to compounds/materials that can be obtained from biological, i.e. renewable, sources. One example for a compound of formula (III) is bio-based 1,4-pentane diol, typically obtained from levulinic acid, which is derived from the degradation of cellulose. An alternative route would be via furfural, furfuryl alcohol, levulinic acid to 1,4-pentane diole.

In one embodiment of the method according to the invention, vinyl ether(s) according to the invention are prepared from bio-based materials, preferably from bio-based diols, most preferably from bio-based 1,4-pentanediol. In various embodiments, compounds of formula (III) are bio-based diols, most preferably bio-based 1,4-pentanediol.

Typically, the term "C₁-C₁₀ alkyl" comprises linear and branched alkyl groups, wherein linear alkyl groups comprise 1 to 10, preferably 1 to 6 C-atoms and branched alkyl groups comprise 3 to 10, preferably 3 to 6 C-atoms. In addition, the term "C₂-C₁₀ alkenyl" comprises linear and branched alkenyl groups, wherein linear alkenyl groups comprise 2 to 10, preferably 2 to 6 C-atoms and branched alkenyl groups comprise 4 to 10, preferably 4 to 6 C-atoms.

In general, in substituted alkyl or alkenyl groups, each H can be independently substituted with a halogen group (e.g. -F, -CI, -Br or- I), -O-CH₃, -O-CH₂-CH₃, or -NO₂. It is however preferred that in various embodiments these groups are unsubstituted.

In various embodiments, in vinyl ether compounds of formula (I)
n is 1; and/or
R₁ is -OH or -O-CH=CH₂; and/or
R₂ is -CH₃; and/or
R₃ is -H; and/or
R₄ is -OH or -O-CH=CH₂; and/or
R₅ is -H;
wherein at least one of R₁ and R₄ is -O-CH=CH₂, preferably R₁ and R₄ are -O-CH=CH₂; and/or
wherein R₆ in the compound of formula (II) is an unsubstituted C₁-C₆ alkyl group, preferably an unsubstituted C₂ alkyl group; and/or
wherein in formula (III)
n is 1; and/or
R₇ is -OH; and/or
R₈ is -CH₃; and/or
R₉ is -H; and/or
R₁₀ is -H; and/or
R₁₁ is -OH.

In various embodiments of the method according to the invention, the compound of formula (II) is added in molar excess over the compound of formula (III), preferably in a molar equivalent ratio of at least 5:1, more preferably of at least 10:1, more preferably of at least 15:1 (formula (II):formula (III)).

In various embodiments of the method according to the invention:
(i) the catalyst is selected from Pd catalysts without being limited to these, preferably Pd(acac)₂, Pd(dba)₂, Pd(OAc)₂, Pd(NO₃)₂ * x H₂O, Pd(PPh₃)₄, PdCl₂(PPh₃)₂, Pd(pivalate) or Pd(II) trifluoroacetate, more preferably Pd(II) trifluoroacetate; and/or
(ii) the bidentate ligand is selected from phenanthrolines and bipyridines without being limited to these, preferably 2,2'-bipyridine; and/or
(iii) the base is a tertiary amine, preferably triethylamine.

In various embodiments, the catalyst is added in amounts of 0.01 to 2 mol%, preferably in 0.1 to 1 mol%, based on the amount of the alcohol of formula (III).

In various embodiments, the bidentate ligand is added in amounts of 0.01 to 2 mol%, preferably in 0.1 to 1 mol%, based on the amount of the alcohol of formula (III).

In various embodiments, the base is added in amounts of 0.1 to 10 mol%, preferably 1 to 5 mol%, more preferably 1.5 to 3.5 mol%, based on the amount of the alcohol of formula (III).

In various embodiments, the method according to the invention is carried out (i) in the presence of at least one solvent or solvent mixture, preferably a polar solvent or solvent mixture, more preferably tetrahydrofuran (THF), ethyl acetate (EtOAc), acetone, 1,4-dioxane or acetonitrile or a mixture thereof. The polar solvent can increase the speed of the reaction due to the improved solubility. In another embodiment, the method according to the invention is carried out in the absence of any additional solvent. In general, this means that no additional compounds, but the compound of formula (II), the compound of formula (III), the catalyst and optionally the bidentate ligand and/or the base and optionally minor amounts of impurities are comprised in the reaction mixture to carry out the method according to the invention to produce a vinyl ether compound of formula (I), wherein the compounds of formula (I), formula (II), formula (III), the catalyst, the bidentate ligand and the base are as defined above.

In some embodiments, the method according to the invention is carried in the presence of a Pd(II) trifluoroacetate (Pd(OOCCF₃)) catalyst, 2,2'-bipyridine and triethylamine, in the absence of any additional solvent.

In various embodiments of the method according to the invention, the reaction temperature is between 20 to 90 °C, preferably between 40 to 80°C or 30 to 60 °C. In specific, non-limiting embodiments according to the invention, the reaction temperature is room temperature or 80 °C.

In various embodiments, the reaction time is 10 to 72 h, preferably 15 to 50 h. Typically, the reaction time can be reduced by increasing the temperature during the course of the reaction.

In various embodiments of the method according to the invention, the vinyl ether compound of formula (I) is a mixture of divinyl ether and mono vinyl ether compounds, preferably the mixture comprises at least 50 wt.-% divinyl ether, more preferably at least 60 wt.-%, more preferably at least 70 wt.-%, most preferably at least 80 wt.-%, based on the total weight of the mixture.

In various embodiments of the method according to the invention, the vinyl ether compound of formula (I) is a mixture of divinyl ether and mono vinyl ether compounds, wherein the ratio of the primary mono vinyl ether compound(s) to the secondary mono vinyl ether compound(s) is at least 60 wt-%, preferably at least 70 wt-%, more preferably at least 80 wt-%, more preferably at least 90 wt-%, more preferably at least 95 wt-% based on the amount of the primary mono vinyl ether compound(s) and the secondary mono vinyl ether compound(s) in the mixture.

Generally, a higher reaction temperature leads to a higher yield of divinyl ether in comparison to mono vinyl ether. A lower temperature generally leads to a higher selectivity of the mixture of mono vinyl ether, i.e. of primary and secondary mono vinyl ether.

In various embodiments of the method according to the invention, the method comprises a further step of product purification. Examples include, without limitation filtration, column chromatography, vacuum evaporation/distillation, with evaporation/distillation being preferred. These steps are commonly known to the skilled person in the art.

According to the second aspect, the present invention relates to a method for producing at least one vinyl ether compound of formula (I) 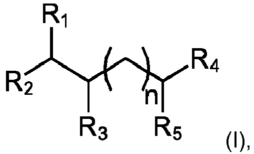 wherein
n is an integer of 0 to 5, preferably 1, 2 or 3, most preferably 1;
R₁, R₃ and R₄ are independently selected from -OH, -O-CH=CH₂, -H or a substituted or unsubstituted C₁-C₁₀ alkyl or C₂-C₁₀ alkenyl group; and
R₂ and R₅ are independently selected from -H or a substituted or unsubstituted C₁-C₁₀ alkyl or C₂-C₁₀ alkenyl group;
with the proviso that at least two of R₁, R₃ and R₄ are -OH or -O-CH=CH₂, and wherein at least one of R₁, R₃ and R₄ is -O-CH=CH₂, preferably R₁ and/or R₄ is/are -O-CH=CH₂;
wherein the method comprises or consists of the following steps:
reacting a compound of formula (III) 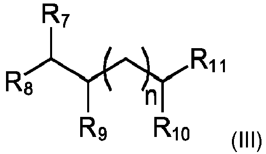 wherein
n is an integer of 0 to 5, preferably 1, 2 or 3, most preferably 1;
R₇, R₉ and R₁₁ are independently selected from -OH, -H or a substituted or unsubstituted C₁-C₁₀ alkyl or C₂-C₁₀ alkenyl group; and
R₈ and R₁₀ are independently selected from -H or a substituted or unsubstituted C₁-C₁₀ alkyl or C₂-C₁₀ alkenyl group;
with the proviso that at least two of R₇, R₉ and R₁₁ are -OH, more preferably R₇ and R₁₁ are -OH;
with
(i) acetylene in the presence of a catalyst and under pressure, preferably 2 to 300 bar, more preferably 2.5 to 60 or 2.5 to 10 bar;
   wherein preferably the catalyst is selected from the group of transition metal acetylides, e.g. a copper acetylide, metal carbonyles, metal carbonyl hydrides, (alkaline (earth)) metal alkoxides or (alkaline (earth)) metal hydroxides or oxides, e.g. potassium hydroxide on activated carbon or magnesium oxide/calcium oxide, without being limited to these; and/or
   wherein preferably the vinylation can be carried out in liquid phase or gas phase; or
(ii) calcium carbide, water and a base, preferably KOH and/or KF,
wherein the reaction is preferably carried out in a polar solvent (mixture), more preferably acetonitrile, 1,4-dioxane, DMF and DMSO.

In some embodiments of the method of aspect 2 according to the invention, in the compound of formula (I)
n is 1; and/or
R₁ is -OH or -O-CH=CH₂; and/or
R2 is -CH₃; and/or
R₃ is -H; and/or
R₄ is -OH or -O-CH=CH₂; and/or
R₅ is -H;
wherein at least one of R₁ and R₄ is -O-CH=CH₂, preferably R₁ and R₄ are -O-CH=CH₂; and/or
wherein in formula (III)
n is 1; and/or
R₇ is -OH; and/or
R₈ is -CH₃; and/or
R₉ is -H; and/or
R₁₀ is -H; and/or
R₁₁ is -OH.

In various embodiments, the compound of formula (III) is bio-based.

According to the third aspect, the present invention relates to a vinyl ether compound of formula (I) 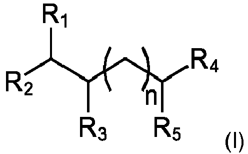 wherein
n is an integer of 0 to 5, preferably 1, 2 or 3, most preferably 1;
R₁, R₃ and R₄ are independently selected from -OH, -O-CH=CH₂, -H or a substituted or unsubstituted C₁-C₁₀ alkyl or C₂-C₁₀ alkenyl group; and
R₂ and R₈ are independently selected from -H or a substituted or unsubstituted C₁-C₁₀ alkyl or C₂-C₁₀ alkenyl group;
with the proviso that at least two of R₁, R₃ and R₄ are -OH or -O-CH=CH₂, and wherein at least one of R₁, R₃ and R₄ is -O-CH=CH₂, preferably R₁ and/or R₄ is/are -O-CH=CH₂;
preferably obtained by a method according to the invention.

In an embodiment of the methods according to the present invention, the compound of formula (I) is a vinyl ether compound of formula (IV) 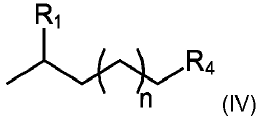 wherein
n is 0, 1, 2, 3, 4 or 5, preferably 1, 2 or 3, most preferably 1;
R₁ and R₄ are -OH or -O-CH=CH₂, with the proviso that at least one of R₁ and R₄ is -O-CH=CH₂, preferably R₁ and R₄ are -O-CH=CH₂; or
the vinyl ether compound is any one of the compounds of formulae (V) to (VII) 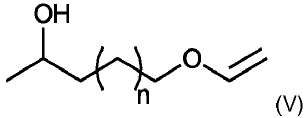 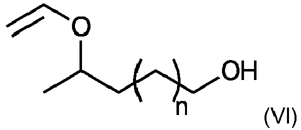 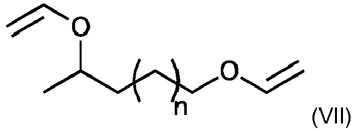 or a mixture thereof;
wherein
n is 0, 1, 2, 3, 4 or 5, preferably 1, 2 or 3, most preferably 1.

In various embodiments, the vinyl ether compound of formula (I) or (IV) according to the invention is a mixture of divinyl ether and mono vinyl ether compounds, preferably the mixture comprises at least 50 wt.-% divinyl ether, more preferably at least 60 wt.-%, more preferably at least 70 wt.-%, most preferably at least 80 wt.-%, based on the total weight of the mixture.

In various embodiments, the vinyl ether compound of formula (I) or (IV) is a mixture of divinyl ether and mono vinyl ether compounds, wherein the ratio of the primary mono vinyl ether compound(s) to the secondary mono vinyl ether compound(s) is at least 60 wt-%, preferably at least 70 wt-%, more preferably at least 80 wt-%, more preferably at least 90 wt-%, more preferably at least 95 wt-% based on the amount of the primary mono vinyl ether compound(s) and the secondary mono vinyl ether compound(s) in the mixture.

According to the fourth aspect, the invention relates to a polymer obtainable by polymerizing a vinyl ether compound of formula (I) according to the invention optionally together with at least one comonomer. Generally, all commonly known comonomers, which can undergo a reaction with a vinyl group are suitable. While cationic polymerization is preferred, thiol-ene reaction and polymerization is similarly possible. Radical polymerization is relevant for vinyl ether-based dispersions or when using vinyl ether monomers as comonomers in radical polymerization with (meth)acrylates

The at least one comonomer may be a further vinyl ether different from formula (I), e.g. an alkyl vinyl ether or a hydroxy(alkyl) vinyl ether, and/or vinyl lactam and/or vinyl amid and/or isocyanate terminated polyol and/or isocyanate terminated polyol endcapped with hydroxyalkyl vinyl ether, more preferably the at least one comonomer is selected from the group of methyl vinyl ether, ethyl vinyl ether, allyl vinyl ether, propyl vinyl ether, isopropyl vinyl ether, butyl vinyl ether, isobutyl vinyl ether, *tert*-butyl vinyl ether, 2-ethylhexyl vinyl ether (EHVE), hydroxyethyl vinyl ether (HEVE), hydroxybutyl vinyl ether or ethoxylated hydroxybutyl vinyl ether with 20-125 EO-units, aminopropyl vinyl ether, phenyl vinyl ether, benzyl vinyl ether, cyclohexyl vinyl ether, butandiol divinyl ether, diethyleneglycol divinyl ether, tri(ethylene glycol) methyl vinyl ether, triethylenglycol divinyl ether, dodecyl vinyl ether, octadecyl vinyl ether, cyclohexane dimethanol divinyl ether (CHDVE), cyclohexane dimethanol mono vinyl ether (CHMVE), trimethylolpropane trivinyl ether (TMPTVE), N-vinyl formamide, N-vinyl pyrrolidone, N-vinyl caprolactame (NVC) or liquid N-vinyl caprolactame (LNVC), N,N'-divinyl-2-imidazolidone, N-vinylimidazole, vinylcarbazol, or isocyanate terminated polyols or isocyanate terminated polyols that are further endcapped with hydroxyalkyl vinyl ether derived from toluene-2,4-diisocyanate (TDI), diphenylmethane diisocyanate (MDI), hexamethylene diisocyanate (HMDI), isophorone diisocyanate (IPDI), pentamethylene diisocyanate (PDI), dicyclohexylmethane-4,4'-diisocyanate (H12MDI), *meta-*tetramethylxylylene diisocyanate (TMXDI) or polymeric diphenylmethane diisocyanate (PMDI).

In various embodiments, more than one co-monomer is present, preferably 2, 3, 4, 5, 6, 7, 8, 9, 10 or more, preferably selected from the list above, but not limited thereto.

Generally, all polymerization techniques, in which a vinyl group can undergo a polymerization reaction are suitable. For example, the mixture of monomers may be polymerized by free radical polymerization to form a vinyl ether polymer or copolymer, without being limited to these methods. Exemplary polymerization initiators include organic azo or peroxo compounds. Suitable polymerization initiators are widely known in the art and readily available. In various embodiments, the polymerization initiator comprises or consists of 2,2'-azobisisobutyronitrile (AIBN). Other suitable initiators include redox initiators, which are widely used and well-known in the art. In various preferred embodiments, the polymerization occurs by cationic polymerization or thiol-ene addition reaction and polymerization. Suitable cationic initiators are known to those skilled in the art and include, without limitation, Brønstedt acids, Lewis acids or super acid generators, such as iodonium or sulfonium salts, which can optionally be activated by UV irradiation.

The polymerization reaction can be carried out by using the vinyl ether compound(s) of formula (I) as defined above, and adding a cationic and/or radical initiator, as detailed above. Additional monomers that can optionally be added, such as further co-monomers, can be added during the polymerization process.

The polymerization can be carried out at elevated temperature, preferably at about 70°C, for several hours, preferably 1 to 12 hours.

After the polymerization is complete, the solvent can be removed by applying heat and/or vacuum to the mixture. Different heating temperatures and/or vacuum settings are suitable depending for example, on the used solvent. In various embodiments, the solvent is evaporated such that residual solvent content in the copolymer is between about 1 wt.-% to 35 wt.-%, preferably about 20 to 25 wt.-% relative to the reaction mixture.

After the polymerization is complete, remaining free monomers can be removed by applying heat and/or vacuum to the mixture. For this, suitable heating temperatures and/or vacuum settings can be applied depending on the type of monomer used, with such conditions being easily determinable for those skilled in the art.

The correspondingly obtained polymers can be used in adhesives, preferably UV adhesives. Such adhesive formulations can additionally contain one or more further components, which are commonly known to those skilled in the art. In specific embodiments, these additional components are selected from fillers, thickeners, silane additives, colorants, perfumes, preservatives, resins and mixtures thereof.

Therefore, according to the fifth aspect, the present invention relates to an adhesive comprising at least one polymer according to the invention.

In various embodiments, the adhesive is an UV adhesive.

Finally in a sixth aspect, the present invention relates to the use of at least one vinyl ether compound of formula (I) according to the invention or at least one polymer according to the invention for the production of UV adhesives, cationic curings, or 1-component or 2-component systems, e.g. can coatings, epoxy- and acrylate-based coatings, structural adhesives, sealants, battery adhesives, wood coatings or pressure sensitive adhesives (PSA)).

All embodiments and examples described above for the method according to the invention may also apply to other aspects according to the invention, and vice versa.

In the following, the invention is described in more detail by reference to the examples. It is understood that these examples are for illustrative purposes only and that it is not intended that the invention is limited thereto.

### EXAMPLES

### Example 1:

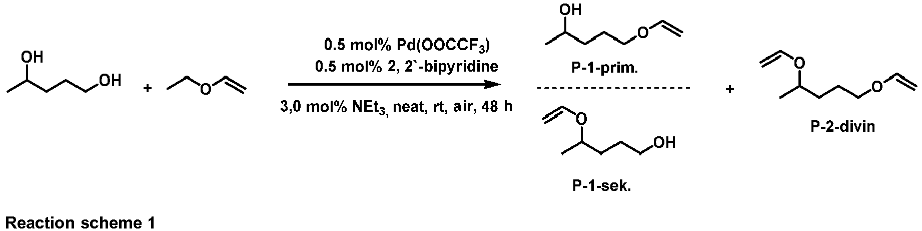

### Vinylation at room temperature

A round bottom flask (1000 mL) was charged with (0.5 mol%; 638.4 mg) Pd(OOCCF₃) and (0.5 mol%; 300 mg) 2,2'-bipyridine. Subsequently, ethyl vinyl ether (15 eq.; 415 mL) and (1.6 mL; 3 mol%) NEt₃ were added. The mixture was allowed to stir for 10 min. Thereafter (40 g; 0.384 mol) 1,4-pentanediol was added. The flask was sealed and stirred at room temperature during the course of the reaction (48 h). The catalyst was filtered off by using a pleated filter whereupon the excess of ethyl vinyl ether was distilled off from the resulting filtrate on a rotary evaporator (40 °C, 700 mbar → 300 mbar). The crude mixture was then distilled under vacuum in order to obtain the first fraction at 45 °C and 0.5 mbar (exclusively **P-2-divin**) and the second fraction at 60 °C and 0.1 mbar (mixture of **P-1-prim.** and **P-1-sek.)**
Conversion 1,4-Pentanediol: 96%
Yield **P-2-divin:** 57%
Yield **P-1-prim.** + **P-1-sek.:** 35% + 1.4%

### Vinylation at 80 °C

A round bottom flask (1000 mL) equipped with a condenser was charged with (0.5 mol%; 638.4 mg) Pd(OOCCF₃) and (0.5 mol%; 300 mg) 2,2'-bipyridine. Subsequently, ethyl vinyl ether (15 eq.; 415 mL) and (1.6 mL; 3 mol%) NEt₃ were added. The mixture was allowed to stir for 10 min at room temperature. Thereafter (40 g; 0.384 mol) 1,4-pentanediol was added. The mixture was stirred at 80 °C during the course of the reaction (16 h). The catalyst was filtered off by using a pleated filter whereupon the excess of ethyl vinyl ether was distilled off from the resulting filtrate on a rotary evaporator (40 °C, 700 mbar → 300 mbar). The crude mixture was then distilled under vacuum in order to obtain the first fraction at 45 °C and 0.5 mbar (exclusively **P-2-divin**) and the second fraction at 60 °C and 0.1 mbar (mixture of **P-1-prim.** and **P-1-sek.**)
Conversion 1,4-Pentanediol: 98%
Yield **P-2-divin:** 72%
Yield **P-1-prim.** + **P-1-sek.:** 15% + 3%

### Isolation of pure mono vinyl ether P-1-prim.

By using column chromatography on silica gel with diethyl ether/n-pentane (1:4) as eluent, the single compound **P-1-prim.** was isolated from the mixture **(P-1-prim./P-1-sek.)** and collected as a yellowish liquid. After applying vacuum distillation as previously described the pure mono vinyl ether **P-1-prim.** was then obtained as a colorless and odorless liquid. **P-1-sek.** underwent self-cyclization during the separation process of column chromatography.

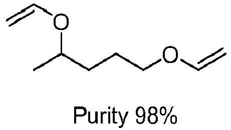

### NMR-Data (in D-Chloroform)

**¹H-NMR** (300 MHz, CDCl₃): 6.44 (dd, 1H, ³J = 6.74 Hz, ³J = 14.33 Hz); 6.29 (dd, 1H, ³J = 6.60 Hz, ³J = 14.12 Hz); 4.24 (dd, 1H, ³J = 14.12 Hz, ²J = 1.53 Hz); 4.14 (dd, 1H, ³J = 14.33 Hz, ²J = 1.95 Hz); 3.96 (dt, 2H, ³J = 6.64 Hz); 3.93-3.86 (m, 1H); 3.70-3.63 (m, 2H); 1.80-1.57 (m, 4H); 1.20 (d, 3H, ³J = 6.20 Hz).
**¹³C-NMR** (75 MHz, CDCl₃): 151.9; 150.9; 88.1; 86.4; 75.4; 67.8; 32.9; 25.1; 19.9 (see Fig. 1).

### GC-MS

**(m/z):** 127; 112; 99; 84; 69; 57; 43; 41; 31.

### IR-Data (ATR-Method)

**ATR-IR** (cm⁻¹): v(C-H, sp²) 3117; v(C-H, sp³) 2973; v(C-H, sp³) 2932; v(C-H, sp³) 2874; (C=C) 1633; (C=C) 1612; (=C-O-C) 1193.

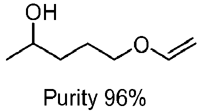

### NMR-Data (in D-Toluene)

**¹H-NMR** (300 MHz, d-toluene): 6.37 (dd, 1H, ³J = 6.84 Hz, ³J = 14.42 Hz); 4.13 (dd, 1H, ³J = 14.42 Hz, ²J = 1.71 Hz); 3.92 (dd, 1H, ³J = 6.84 Hz, ²J = 1.71 Hz); 3.53 (sextett, 1H, ³J = 6.21 Hz); 3.43 (td, 2H, ⁴J = 1.44 Hz, ³J = 6.32 Hz); 2.18 (br, 1H); 1.68-1.45 (m, 2H); 1.35-1.26 (m, 2H); 1.00 (d, 3H, ³J = 6.20 Hz). **¹³C-NMR** (75 MHz, d-toluene): 152.3; 86.2; 68.0; 67.4; 36.0; 25.8; 23.7.

### GC-MS

(m/z): 130, 115, 102, 87, 69, 58, 45, 43, 31.

### IR-Data (ATR-Method)

**ATR-IR (cm⁻¹):** v(O-H) 3362; v(C-H, sp²) 3118; v(C-H, sp³) 2964; v(C-H, sp³) 2928; v(C-H, sp³) 2873; (C=C) 1636; (C=C) 1613; (=C-O-C) 1197.

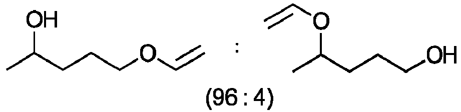

### NMR-Data (in D-Toluene)

**¹H-NMR** (300 MHz, d-toluene): 6.37 (dd, **1H,** ³J = 6.84 Hz, ³J = 14.34 Hz); 6.15 (dd, **0.04H,** ³J = 6.60 Hz, ³J = 14.14 Hz); 4.29 (dd, **0.04H,** ³J = 14.14 Hz, ²J = 1.33 Hz); 4.13 (dd, **1H, ³J** = 14.34 Hz, ²J = 1.78 Hz); 3.94 (dd, **0.04H,** ³J = 6.60 Hz, ²J = 1.33 Hz); 3.92 (dd, **1H**, ³J = 6.83 Hz, ²J = 1.78 Hz); 3.60-3.48 (m, **1H**, **0.04H** ); 3.43 (td, **2H, 0.08H** ⁴J = 1.40 Hz, ³J = 6.34 Hz); 2.20 (d, **1H,** ³J = 4.64 Hz); 2.17 (br, **0.04H**); 1.67-1.45 (m, **2H, 0.08H**); 1.35-1.23 (m, **2H, 0.08H**); 1.02 (d, **0.12H,** ³J = 6.19 Hz); 1.00 (d, **3H,** ³J = 6.20 Hz).
**¹³C-NMR** (75 MHz, d-toluene): **152.3; 151.3; 88.0; 86.2; 75.6; 68.0; 67.4; 62.3; 36.0; 33.1; 29.0; 25.8; 23.7; 19.9.**

## Claims

1. A method for producing at least one vinyl ether compound of formula (I) 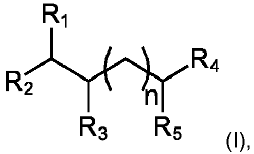 wherein
n is an integer of 0 to 5, preferably 1, 2 or 3, most preferably 1;
R₁, R₃ and R₄ are independently selected from -OH, -O-CH=CH₂, -H or a substituted or unsubstituted C₁-C₁₀ alkyl or C₂-C₁₀ alkenyl group; and
R₂ and R₅ are independently selected from -H or a substituted or unsubstituted C₁-C₁₀ alkyl or C₂-C10 alkenyl group;
with the proviso that at least two of R₁, R₃ and R₄ are -OH or -O-CH=CH₂, and wherein at least one of R₁, R₃ and R₄ is -O-CH=CH₂, preferably R₁ and/or R₄ is/are -O-CH=CH₂;
wherein the method comprises or consists of the following steps:
reacting a compound of formula (II) 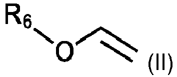 wherein R₆ is -C(=O)R₁₂, or an unsubstituted C₁-C₁₀ alkyl or C₂-C₁₀ alkenyl group, preferably an unsubstituted C₁-C₆ alkyl group, most preferably an unsubstituted C₂ alkyl group; and
R₁₂ is an unsubstituted C₁-C₁₀ alkyl group, preferably -CH₃,
with a compound of formula (III) 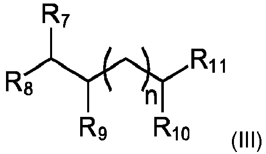 wherein
n is an integer of 0 to 5, preferably 1, 2 or 3, most preferably 1;
R₇, R₉ and R₁₁ are independently selected from -OH, -H or a substituted or unsubstituted C₁-C₁₀ alkyl or C₂-C₁₀ alkenyl group; and
R₈ and R₁₀ are independently selected from -H or a substituted or unsubstituted C₁-C₁₀ alkyl or C₂-C₁₀ alkenyl group;
with the proviso that at least two of R₇, R₉ and R₁₁ are -OH, preferably R₇ and R₁₁ are -OH;
in the presence of a catalyst and optionally a bidentate ligand and/or a base.

2. The method of claim 1, wherein in formula (I)
n is 1; and/or
R₁ is -OH or -O-CH=CH₂; and/or
R2 is -CH₃; and/or
R3 is -H; and/or
R₄ is -OH or -O-CH=CH₂; and/or
R₅ is -H;
wherein at least one of R₁ and R₄ is -O-CH=CH₂, preferably R₁ and R₄ are -O-CH=CH₂; and/or
wherein R₆ in the compound of formula (II) is an unsubstituted C₂ alkyl group; and/or
wherein in formula (III)
n is 1; and/or
R₇ is -OH; and/or
R₈ is -CH₃; and/or
R₉ is -H; and/or
R₁₀ is -H; and/or
R₁₁ is -OH.

3. The method of claim 1 or 2, wherein the reaction temperature is between 20 to 90 °C.

4. The method of any one of claims 1 to 3, wherein
(i) the catalyst is selected from Pd catalysts, preferably Pd(acac)₂, Pd(dba)₂, Pd(OAc)₂, Pd(NO₃)₂* xH₂O, Pd(PPh₃)₄, PdCl₂(PPh₃)₂, Pd(pivalate) or Pd(II) trifluoroacetate, more preferably Pd(II) trifluoroacetate; and/or
(ii) the bidentate ligand is selected from phenanthrolines and bipyridines, preferable 2,2'-bipyridine; and/or
(iii) the base is a tertiary amine, preferably triethylamine.

5. The method of any one of claims 1 to 4, wherein the method is carried out
(i) in the presence of at least one solvent, preferably a polar solvent, more preferably tetrahydrofuran (THF), ethyl acetate (EtOAc), acetone, 1,4-dioxane or acetonitrile; or
(ii) in the absence of any additional solvent; and/or
(ii) under oxygen or air.

6. The method of any one of claims 1 to 5, wherein
the compound of formula (II) is added in molar excess over the compound of formula (III), preferably in a molar equivalent ratio of at least 1:1 or >1:1 (formula (II) : formula (III)).

7. The method of any one of claims 1 to 6, wherein the vinyl ether compound of formula (I) is a mixture of divinyl ether and mono vinyl ether compounds, preferably the mixture comprises at least 50 wt.-% divinyl ether, more preferably at least 60 wt.-%, more preferably at least 70 wt.-%, most preferably at least 80 wt.-%, based on the total weight of the mixture.

8. A method for producing at least one vinyl ether compound of formula (I) 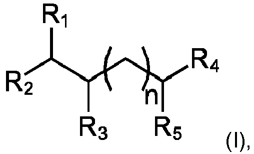 wherein
n is an integer of 0 to 5, preferably 1, 2 or 3, most preferably 1;
R₁, R₃ and R₄ are independently selected from -OH, -O-CH=CH₂, -H or a substituted or unsubstituted C₁-C₁₀ alkyl or C₂-C₁₀ alkenyl group; and
R₂ and R₅ are independently selected from -H or a substituted or unsubstituted C₁-C₁₀ alkyl or C₂-C₁₀ alkenyl group;
with the proviso that at least two of R₁, R₃ and R₄ are -OH or -O-CH=CH₂, and wherein at least
one of R₁, R₃ and R₄ is -O-CH=CH₂, preferably R₁ and/or R₄ is/are -O-CH=CH₂;
wherein the method comprises or consists of the following steps:
reacting a compound of formula (III) 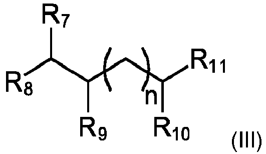 wherein
n is an integer of 0 to 5, preferably 1, 2 or 3, most preferably 1;
R₇, R₉ and R₁₁ are independently selected from -OH, -H or a substituted or unsubstituted C₁-C₁₀ alkyl or C₂-C₁₀ alkenyl group; and
R₈ and R₁₀ are independently selected from -H or a substituted or unsubstituted C₁-C₁₀ alkyl or C₂-C₁₀ alkenyl group;
with the proviso that at least two of R₇, R₉ and R₁₁ are -OH, more preferably R₇ and R₁₁ are -OH;
with
(i) acetylene in the presence of a catalyst and under pressure,
wherein preferably the catalyst is selected from the group of transition metal acetylides, e.g. a copper acetylide, metal carbonyles, metal carbonyl hydrides, (alkaline (earth)) metal alkoxides or (alkaline (earth)) metal hydroxides or oxides, e.g. potassium hydroxide on activated carbon or magnesium oxide/calcium oxide; and/or
wherein preferably the vinylization can be carried out in liquid phase or gas phase; or
(ii) calcium carbide, water and a base, preferably KOH and/or KF,
wherein the reaction is preferably carried out in a solvent (mixture), more preferably water and DMSO.

9. The method of claim 8, wherein in formula (I)
n is 1; and/or
R₁ is -OH or -O-CH=CH₂; and/or
R2 is -CH₃; and/or
R3 is -H; and/or
R₄ is -OH or -O-CH=CH₂; and/or
R₅ is -H;
wherein at least one of R₁ and R₄ is -O-CH=CH₂, preferably R₁ and R₄ are -O-CH=CH₂; and/or
wherein in formula (III)
n is 1; and/or
R₇ is -OH; and/or
R₈ is -CH₃; and/or
R₉ is -H; and/or
R₁₀ is -H; and/or
R₁₁ is -OH.

10. The method of any one of claims 1 to 9, wherein the compound of formula (III) is bio-based.

11. A vinyl ether compound of formula (I) 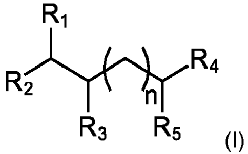 wherein
n is an integer of 0 to 5, preferably 1, 2 or 3, most preferably 1;
R₁, R₃ and R₄ are independently selected from -OH, -O-CH=CH₂, -H or a substituted or unsubstituted C₁-C₁₀ alkyl or C₂-C₁₀ alkenyl group; and
R₂ and R₈ are independently selected from -H or a substituted or unsubstituted C₁-C₁₀ alkyl or C₂-C₁₀ alkenyl group;
with the proviso that at least two of R₁, R₃ and R₄ are -OH or -O-CH=CH₂, and wherein at least one of R₁, R₃ and R₄ is -O-CH=CH₂, preferably R₁ and/or R₄ is/are -O-CH=CH₂;
preferably obtained by a method according to any of claims 1 to 10.

12. The compound of claim 11, wherein the vinyl ether compound is of formula (IV) 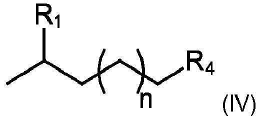 wherein
n is 0, 1, 2, 3, 4 or 5, preferably 1, 2 or 3, most preferably 1;
R₁ and R₄ are -OH or -O-CH=CH₂, with the proviso that at least one of R₁ and R₄ is -O-CH=CH₂, preferably R₁ and R₄ are -O-CH=CH₂; or
the vinyl ether compound is any one of the compounds of formulae (V) to (VII) 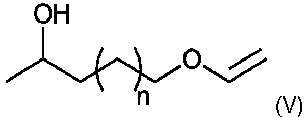 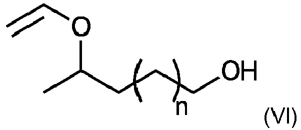 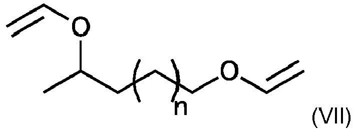 or a mixture thereof;
wherein
n is 0, 1, 2, 3, 4 or 5, preferably 1, 2 or 3, most preferably 1.

13. A polymer obtainable by polymerizing a compound of claim 11 or 12 optionally together with at least one comonomer.

14. An adhesive comprising at least one polymer according to claim 13.

15. Use of at least one compound of claim 11 or 12 or at least one polymer of claim 13 for the production of UV adhesives, cationic curings, or 1-component or 2-component systems, e.g. can coatings, epoxy- and acrylate-based coatings, structural adhesives, sealants, battery adhesives, wood coatings or pressure sensitive adhesives (PSA)).
